# EUROPÄISCHE PATENTSCHRIFT

(11) **EP 2 032 518 B1**
(45) Veröffentlichungstag und Bekanntmachung des Hinweises auf die Patenterteilung: **26.05.2010**
(21) Anmeldenummer: 07712526.8
(22) Anmeldetag: 14.03.2007
(51) Int. Cl.: C07C 57/04

(54) **VERFAHREN ZUR KONTINUIERLICHEN HERSTELLUNG VON UNGESÄTTIGTEN CARBONSÄUREANHYDRIDEN**
METHOD FOR THE CONTINUOUS PRODUCTION OF UNSATURATED CARBOXYLIC ACID ANHYDRIDES
PROCÉDÉ DE PRODUCTION EN CONTINU D'ANHYDRIDES D'ACIDES CARBOXYLIQUES INSATURÉS

(30) Priorität: 23.06.2006 DE 102006029320
(43) Veröffentlichungstag der Anmeldung: 11.03.2009
(73) Patentinhaber: Evonik Röhm GmbH, 64293 Darmstadt (DE)
(72) Erfinder: BRÖLL, Dirk, 63225 Langen (DE); SIEGERT, Hermann, 64342 Seeheim-Jugenheim (DE)
(86) Internationale Anmeldenummer: PCT/EP2007/052398
(87) Internationale Veröffentlichungsnummer: WO 2007/147652

(56) Entgegenhaltungen:
- EP-A- 0 004 641
- EP-A- 0 196 520
- FR-A1- 2 877 003
- US-A- 4 857 239

## Beschreibung

Die Erfindung beschreibt ein Verfahren zur kontinuierlichen Herstellung von ungesättigten Carbonsäureanhydriden, insbesondere die Reaktion einer ungesättigten Carbonsäure mit einem niedermolekularen aliphatischen Carbonsäureanhydrid.

In DE-A-3510035 wird ein Verfahren zur kontinuierlichen Herstellung von ungesättigten Carbonsäureanhydriden durch säurekatalysierte Umanhydridisierungsreaktion von Essigsäureanhydrid mit einer ungesättigten Carbonsäure im Mittelteil einer Destillationskolonne beschrieben. Zur Erreichung eines vollständigen Umsatzes wird Essigsäureanhydrid in einem Überschuss von 0.1 bis 0.5 Mol pro Mol Carbonsäure eingesetzt, wobei dann am Kolonnenkopf eine Mischung aus Essigsäure und Essigsäureanhydrid anfällt, also keine reine Essigsäure gewonnen wird.

Darüber hinaus wird das Produkt verunreinigt durch den Katalysator gebildet, der erst in einem weiteren Verfahrensschritt entfernt werden muss.

In US-A- 4,857,239 wird ein Verfahren zur Herstellung von Methacrylsäureanhydrid beschrieben, wobei das Molverhältnis von Methacrylsäure zu Essigsäureanhydrid 2.1 bis 3 beträgt und ein Polymerisationsinhibitor in die Destillationskolonne zugegeben wird. Gemäß den Beispielen ist das Verfahren diskontinuierlich. Nachteilig darüber hinaus ist, dass das im Überschuss eingesetzte Edukt ungenutzt anfällt.

In US-A-2003/0018217 wird ein diskontinuierliches Verfahren zur Herstellung von Methacrylsäureanhydrid beschrieben, wobei das anfängliche Molverhältnis von Methacrylsäure zu Essigsäureanhydrid bevorzugt 9 bis 11 beträgt. Die entstehende Essigsäure wird sofort abgeführt und der freiwerdende Reaktorinhalt mit Essigsäureanhydrid aufgefüllt. Zur Vermeidung einer Polymerisation werden in den Reaktor und in die Kolonne Inhibitoren zugegeben. Es werden viele Nebenprodukte gebildet, die sich nicht vollständig entfernen lassen.

Aufgabe ist es nun ein-verbessertes Verfahren zur kontinuierlichen Herstellung von ungesättigten Carbonsäureanhydriden bereitzustellen, bei dem ein stöchiometrischer Überschuss eines der Edukte vermieden wird, trotzdem ein vollständiger Umsatz der Edukte erzielt wird und gleichzeitig das ungesättigte Anhydrid und die gebildete Carbonsäure in hoher Reinheit gewonnen werden. Darüber hinaus soll die Polymerisation in allen Bereichen weitgehend vermieden werden und die Raum-Zeit-Ausbeute der Reaktion erhöht werden.

Gegenstand der Erfindung ist ein Verfahren zur kontinuierlichen Herstellung von ungesättigten Carbonsäureanhydriden der allgemeinen Formel I

R-C(O)-O-C(O)-R (I),

in der R einen ungesättigten organischen Rest mit 2 bis 12 C-Atomen darstellt, durch Umanhydridisierung von einem aliphatischen Carbonsäureanhydrid mit einer Carbonsäure der allgemeinen Formel II

R-COOH (II),

in der R die oben angegebene Bedeutung hat,
in einer Rektifkationskolonne mit einem oberen, mittleren und unteren Bereich, dadurch gekennzeichnet, dass
a) im Sumpf der Kolonne ein inertes Siedeöl vorgelegt ist,
b) die Edukte in stöchiometrischen Verhältnissen in einen Reaktionsbereich eingespeist werden,
c) am Kopf der Kolonne die als Nebenprodukt entstehende Carbonsäure entnommen wird,
d) die nicht umgesetzten Edukte in den Reaktionsbereich zurückgeführt werden und
e) das Produkt der Formel I über einen Seitenabzug, bevorzugt zwischen dem mittleren und unteren Kolonnenbereich, erhalten wird.

Durch diese technischen Merkmale wird erreicht, dass ein vollständiger Umsatz der Edukte und gleichzeitig eine hohe Reinheit der Produkte erzielt wird und die Polymerisation in allen Bereichen weitgehend vermieden wird, da u.a. lange Verweilzeiten des gebildeten ungesättigten Anhydrids im Kolonnensumpf ausgeschlossen werden.

Für das erfindungsgemäße Verfahren geeignete Carbonsäuren weisen einen ungesättigten organischen Rest mit 2 bis 12, vorzugsweise 2 bis 6, besonders bevorzugt 2 bis 4 Kohlenstoffatomen auf. Geeignete Alkenylgruppen sind die Vinyl-, Allyl-, 2-Methyl-2-propen-, 2-Butenyl-, 2-Pentenyl-, 2-Decenyl, 1-Undecenyl und die 9,12-Octadecadienyl-Gruppe. Besonders bevorzugt sind die Vinyl und die Allylgruppe.

Zu den besonders bevorzugten Carbonsäuren gehören u.a. (Meth)acrylsäuren. Der Begriff (Meth)acrylsäuren ist in der Fachwelt bekannt, wobei hierunter neben Acrylsäure und Methacrylsäure auch Derivate dieser Säuren zu verstehen sind. Zu diesen Derivaten gehören unter anderem β-Methylacrylsäure (Butensäure, Crotonsäure), α, β-Dimethylacrylsäure, β-Ethylacrylsäure, α-Chloracrylsäure, α-Cyanacrylsäure, 1-(Trifluormethyl)acrylsäure, sowie β,β-Dimethylacrylsäure. Bevorzugt sind Acrylsäure (Propensäure) und Methacrylsäure (2-Methylpropensäure).

Geeignete Carbonsäureanhydride für das erfindungsgemäße Verfahren sind der Fachwelt ebenfalls bekannt. Bevorzugte Verbindungen besitzen die allgemeine Formel III R'-C(O)-O-C(O)-R' (III), worin R' einen C₁ bis C₄-Alkylrest darstellt. Vorzugsweise wird Essigsäureanhydrid verwendet.

Für das erfindungsgemäße Verfahren wird als Siedeöl eine hochsiedende, inerte thermisch langzeitstabile Substanz mit einem Siedepunkt höher als die Siedepunkte der an der Reaktion beteiligten Komponenten verwendet, um die destillative Abtrennung des gebildeten Säureanhydrids ohne Polymerisation zu gewährleisten. Der Siedepunkt des Siedöls sollte aber auch nicht zu hoch sein, um die thermische Belastung des gebildeten Säureanhydrids zu reduzieren.

Im Allgemeinen liegt die Siedetemperatur des Siedeöls bei Normaldruck (1013 mbar) bei 200 bis 400°C, insbesondere bei 240 bis 290 °C.

Geeignete Siedeöle sind u.a. höherkettige unverzweigte Paraffine mit 12 bis 20 Kohlenstoffatomen, aromatische Verbindungen wie Diphyl (eutektische Mischung aus 75% Biphenyloxid und 25% Biphenyl), alkylsubstituierte Phenole oder Naphthalinverbindungen, Sulfolan (Tetrahydro-thiophen-1,1-dioxid) oder Mischungen aus diesen.

Geeignete Beispiele sind die nachfolgend aufgerührten Siedeöle:

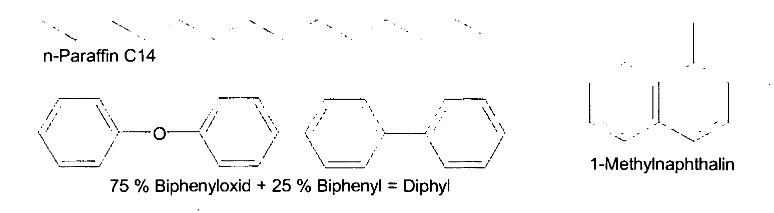

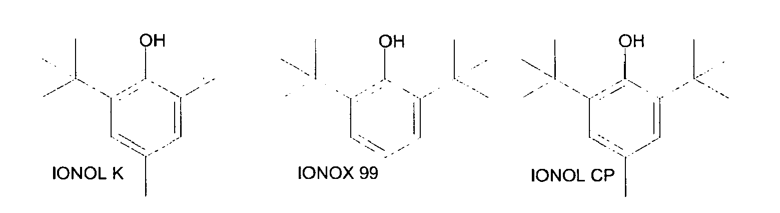

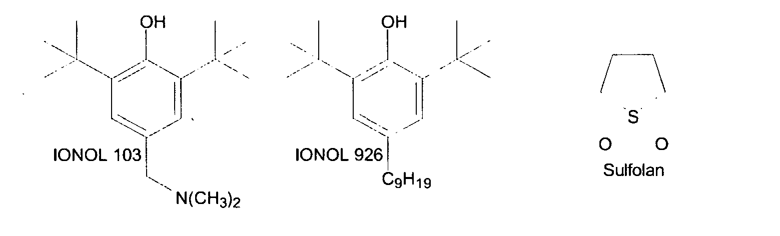

Besonders bevorzugt werden 2,6-di-tert-Butyl-para-cresol, 2,6-di-tert-Butylphenol, Sulfolan, Diphyl oder Mischungen aus diesen eingesetzt, ganz besonders bevorzugt Sulfolan.

Erfindungsgemäß ist unter einem stöchiometrischen Verhältnis ein molares Verhältnis von 1,9 bis 2,1 zu 1 von Carbonsäure zu Carbonsäureanhydrid zu verstehen.

Für die Umanhydridisierungsreaktion gemäß der vorliegenden Erfindung kann jede Rektifikationskolonne verwendet werden, die im oberen, mittleren und unteren Bereich je 5 bis 15 Trennstufen besitzt. Bevorzugt beträgt die Zahl der Trennstufen im oberen Bereich 10 bis 15 und im mittleren und unteren Bereich 8 bis 13. Als Anzahl der Trennstufen wird in der vorliegenden Erfindung die Anzahl der Böden bei einer Bodenkolonne multipliziert mit dem Bodenwirkungsgrad oder die Anzahl der theoretischen Trennstufen im Fall einer Packungskolonne oder eine Kolonne mit Füllkörpern bezeichnet.

Beispiele für eine Rektifikationskolonne mit Böden beinhalten solche wie Glockenböden, Siebböden, Tunnelböden, Ventilböden, Schlitzböden, Sieb-Schlitzböden, Sieb-Glockenböden, Düsenböden, Zentrifugalböden, für eine Rektifikationskolonne mit Füllkörpern solche wie Raschig-Ringe, Lessing-Ringe, Pall-Ringe, Berl-Sättel, Intalox Sättel und für eine Rektifikationskolonne mit Packungen solche wie vom Typ Mellapak (Sulzer), Rombopak (Kühni), Montz-Pak (Montz) und Packungen mit Katalysatortaschen, beispielsweise Katapak (Sulzer).

Eine Rektifikationskolonne mit Kombinationen aus Bereichen von Böden, aus Bereichen von Füllkörper und/oder aus Bereichen von Packungen kann ebenso verwendet werden.

Bevorzugt wird eine Rektifikationskolonne, mit Füllkörpern und/oder Packungen für die 3 Bereiche, eingesetzt.

Die Rektifikationskolonne kann aus jedem hierfür geeigneten Material hergestellt werden. Hierzu gehören u.a. Edelstahl sowie inerte Materialien.

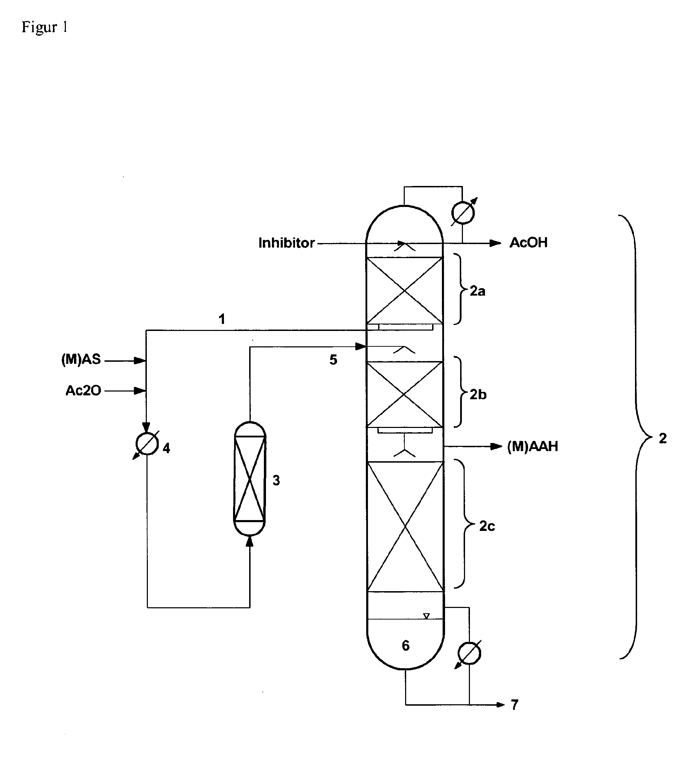

Die Apparatur weist mindestens einen Bereich, nachfolgend Reaktionsbereich oder Reaktor genannt, auf, in dem vorzugsweise mindestens ein Katalysator vorgesehen ist. Dieser Reaktor kann innerhalb und/oder außerhalb der Rektifikationskolonne liegen. Vorzugsweise wird dieser Reaktor jedoch außerhalb der Rektifikationskolonne in einem separaten Bereich angeordnet, wobei eine dieser bevorzugten Ausführungsformen in Figur 1 näher erläutert wird.

Die Reaktion wird bevorzugt bei Temperaturen im Bereich von 30 bis 120 °C, besonders bevorzugt bei 40 bis 100 °C, insbesondere bei 50 bis 80 °C, durchgeführt. Dabei ist die Reaktionstemperatur abhängig vom eingestellten Systemdruck. Bei einer Anordnung des Reaktors innerhalb der Kolonne wird die Reaktion vorzugsweise im Druckbereich von 5 bis 100 mbar (absolut), insbesondere bei 10 bis 50 mbar (absolut) und besonders bevorzugt bei 20 bis 40 mbar (absolut) durchgeführt.

Falls sich der Reaktor außerhalb der Kolonne befindet, können dort andere Druck- und Temperaturverhältnisse gewählt werden als in der Kolonne. Das hat den Vorteil, dass die Reaktionsparameter des Reaktors unabhängig von den Betriebsbedingungen in der Kolonne eingestellt werden können.

Die Reaktionsdauer der Umanhydridisierung hängt von der Reaktionstemperatur ab; die Verweilzeit im Reaktor bei einmaligem Durchgang beträgt vorzugsweise 0,5 bis 15 Minuten und besonders bevorzugt 1 bis 5 Minuten.

Bei der Herstellung von (Meth)acrylsäureanhydrid aus Essigsäureanhydrid und (Meth)acrylsäure beträgt die Reaktionstemperatur vorzugsweise 40 bis 100 °C, besonders bevorzugt 50 bis 90 °C und ganz besonders bevorzugt 70 bis 85 °C.

Die Reaktionsmischung kann neben den Reaktanten weitere Bestandteile, wie beispielsweise Lösungsmittel, Katalysatoren und Polymerisationsinhibitoren umfassen.

Bevorzugt werden in dem Reaktionsbereich heterogene Katalysatoren verwendet. Als heterogene Katalysatoren besonders geeignet sind saure Festbettkatalysatoren, insbesondere saure Ionenaustauscher.

Zu den besonders geeigneten sauren Ionenaustauschern gehören insbesondere Kationenaustauscherharze wie sulfonsäuregruppenhaltige Styrol-Divinylbenzolpolymere. Geeignete Kationenaustauscherharze können kommerziell von Rohm&Haas unter der Handelsbezeichnung Amberlyst^{®}, von Dow unter der Handelsbezeichung Dowex^{®} und von Lanxess unter der Handelsbezeichnung Lewatit^{®} erhalten werden.

Die Katalysatormenge in L beträgt vorzugsweise 1/10 bis das 2fache, besonders bevorzugt 1/5 bis 1/2, der zu produzierenden Menge an neu gebildetem ungesättigten Carbonsäureanhydrid in L/h.

Zu den bevorzugt einsetzbaren Polymerisationsinhibitoren gehören unter anderem Octadecyl-3-(3,5-di-tert-butyl-4-hydroxyphenyl)-propionat, Phenothiazin, Hydrochinon, Hydrochinonmonomethylether, 4-Hydroxy-2,2,6,6-tetramethylpiperidinooxyl (TEMPOL), 2,4-Dimethyl-6-tert-butylphenol, 2,6-Di-tert-butylphenol, 2,6-Di-tert-butyl-4-methylphenol, para-substituierte Phenylendiamine wie z. B. N,N'-Diphenyl-p-phenylendiamin, 1,4-Benzochinon, 2,6-Di-tert-butyl-alpha-(dimethylamino)-p-cresol, 2,5-Di-tert-butylhydrochinon oder Gemische aus zwei oder mehreren dieser Stabilisatoren. Ganz besonders bevorzugt ist Phenothiazin.

Die Zudosierung des Inhibitors kann in den Feed vor den Reaktor und/oder in die Rektifikationskolonne, vorzugsweise an deren Kopf, erfolgen.

Erfindungsgemäß erfolgt die Umanhydridisierung in einer Apparatur, wobei die Feedströme der Edukte mit dem Reaktorkreislaufstrom, der überwiegend aus nicht umgesetzten Edukten sowie einem Zwischenprodukt der Formel R-C(O)-O-C(O)-R', wobei R und R' die obengenannte Bedeutung haben, in den Reaktionsbereich der Rektifikationskolonne eingespeist werden. Im Sumpf der Kolonne befindet sich das obengenannte inerte Siedeöl, um lange Verweilzeiten des polymerisationsanfälligen Zielproduktes zu verkleiden. Das ungesättigte Carbonsäureanhydrid als Zielprodukt wird zwischen dem mittleren und unteren Bereich bevorzugt gasförmig abgezogen, während am Kopf der Kolonne die neu gebildete Carbonsäure als leichtest siedende Reaktionskomponente abgezogen wird. Nicht umgesetzte Edukte und gebildete Zwischenprodukte werden in den Reaktionsbereich zurückführt, zum Beispiel mittels einer Pumpe.

Aus dem Sumpf können Hochsieder wie zugesetzte Inhibitoren durch übliche Methoden ausgeschleust werden, beispielsweise durch einen Dünnschichtverdampfer oder einen ähnliche Aufgaben verrichtenden Apparat, der verdampfende Stoffe in die Rektifikationskolonne zurückführt und nicht verdampfende Hochsieder ausschleust.

Falls ein Katalysator eingesetzt wird, kann dieser in jedem Bereich der Rektifikationskolonne vorgesehen sein, bevorzugt im mittleren Bereich.

Des Weiteren kann der Katalysator in einem separaten Bereich der Apparatur, dem Reaktionsbereich oder Reaktor bereitgestellt werden, wobei dieser Bereich mit den weiteren Bereichen der Apparatur verbunden ist. Diese separate Anordnung des Katalysatorbereichs ist bevorzugt, wobei die Edukte ständig durch den Katalysatorbereich geleitet werden können. Hierdurch entsteht kontinuierlich das ungesättigte Carbonsäureanhydrid, beispielsweise (Meth)acrylsäureanhydrid, sowie die neu gebildete Carbonsäure, beispielsweise Essigsäure.

Eine bevorzugte Ausführungsform des erfindungsgemäßen Verfahrens ist in Fig. 1 schematisch dargelegt.

Die Feedströme von (Meth)acrylsäure (=(M)AS)) und Essigsäureanhydrid (=Ac₂O) werden mit dem Kreislaufstrom (1), der überwiegend aus nicht umgesetzten Edukten sowie dem gebildeten Zwischenprodukt Acetyl(meth)acrylat besteht, einem außerhalb der Rektifikationskolonne (2) gelagerten Reaktor (3) zugeführt.

Die Temperatur der Reaktanten kann dabei über einen Wärmetauscher (4) in der Zuführung eingestellt werden.

Der Reaktor ist vorzugsweise ein Strömungsrohrreaktor, der einen Festbettkatalysator enthält.

Der Reaktorabstrom (5) wird in die Rektifikationskolonne (2) eingespeist, vorzugsweise unterhalb des Rücklaufstromes aus dem oberen Bereich (2a) der Kolonne. In der Kolonne (2) finden zum einen die Weiterreaktion und zum anderen die Trennung der Komponenten statt. Zur Vermeidung von Polymerisation wird vorzugsweise am Kopf der Kolonne Inhibitor zudosiert.

Im oberen Bereich (2a) wird die leichtsiedende Essigsäure von den Mittelsiedern (Edukte, Zwischenprodukt) abgetrennt und am Kopf abgezogen. Im mittleren Bereich (2b) der Kolonne findet die Trennung Mittelsieder gegen (Meth)acrylsäureanhydrid (= (M)AAH) statt, wobei zwischen dem mittleren und unteren Teil (M)AAH bevorzugt gasförmig abgezogen wird. Im unteren Bereich (2c) der Kolonne wird (M)AAH von dem im Sumpf befindlichen Siedeöl (6) getrennt. Im Sumpf befindliche Hochsieder können durch übliche Methoden (7) ausgeschleust werden, beispielsweise durch einen Dünnschichtverdampfer oder einen ähnliche Aufgaben verrichtenden Apparat, der verdampfende Stoffe in die Rektifikationskolonne zurückführt und nicht verdampfende Hochsieder ausschleust.

Der aus dem oberen Bereich (2a) resultierende Flüssigkeitsstrom wird vollständig aus der Kolonne abgezogen und als Kreislaufstrom (1) zusammen mit den Feedströmen dem Reaktor zugeführt.

Nachfolgend soll die vorliegende Erfindung anhand von Beispielen näher erläutert werden.

### Beispiel 1: Herstellung von Methacrylsäureanhydrid

Für die Herstellung von Methacrylsäureanhydrid durch Reaktion von Methacrylsäure und Essigsäureanhydrid wurde eine Versuchsanlage gemäß Figur 1 aufgebaut. Die Rektifikationskolonne (2) hatte insgesamt ca. 35 Trennstufen (15 im oberen Bereich (2a), 12 im mittleren Bereich (2b) und 8 im unteren Bereich (2c)). Diese Kolonne war mit Zwischenstücken und Sumpf 5,5 m hoch, hatte einen Innendurchmesser von 100 mm und war mit Packungen der Fa. Sulzer, Typ CY (Bereich 2a und 2b) und der Fa. Montz, Typ BSH 400 bestückt (Bereich 2c). Als Polymerisationsinhibitor wurde Phenothiazin eingesetzt. Der Druck am Kolonnenkopf betrug 20 mbar. Bei stationären Bedingungen stellte sich ein Temperaturprofil von 164 °C (Sumpf) bis 23 °C (Kolonnenkopf) ein. Die Austräge an Essigsäure am Kolonnenkopf und Methacrylsäureanhydrid am Seitenabzug (zwischen Bereich 2b und 2c) sowie die Heizdampfleistung des Sumpfverdampfers wurden durch Einstellung geeigneter Temperaturen in den jeweiligen Bereichen geregelt.

Im Sumpf der Rektifikationskolonne wurden 6 kg Sulfolan als Siedeöl (6) eingesetzt. Als Verdampfer diente ein Fallfilmverdampfer.

Die Reaktion wurde im außen liegenden Reaktor (3) durchgeführt. Als heterogener Feststoffkatalysator wurden 450 mL des sauren Ionenaustauschers Lewatit K2431 von der Fa. Lanxess verwendet. Die Reaktionstemperatur betrug 76 °C. Der von der Kolonne kommende Kreislaufstrom (1), ca. 16 kg/h, der in erster Linie aus nicht umgesetzten Edukten und dem Zwischenprodukt Acetylmethacrylat bestand, gelangte mit der Frischeinspeisung von Methacrylsäure und Essigsäureanhydrid in den Reaktor zurück.

Es wurden 1750 g/h Essigsäureanhydrid und 2951 g/h Methacrylsäure kontinuierlich frisch zudosiert.

Am Kolonnenkopf fielen 2038 g/h Essigsäure an. Am Seitenstromabzug wurden 2616 g/h Methacrylsäureanhydrid mit einer Reinheit von 99,7 % (GC-Analyse) entnommen. Die Ausbeute an Methacrylsäureanhydrid bezogen auf eingesetztes Essigsäureanhydrid bzw. eingesetzte Methacrylsäure betrug 99 %.

### Beispiel 2: Herstellung von Acrylsäureanhydrid

Für die Herstellung von Acrylsäureanhydrid durch Reaktion von Acrylsäure und Essigsäureanhydrid wurde dieselbe Versuchsanlage wie in Beispiel 1 erläutert verwendet.

Der Druck am Kolonnenkopf, die Reaktionstemperatur und der Kreislaufstrom waren praktisch identisch mit den Werten, die in Beispiel 1 angegeben sind. Ebenfalls wurden dieselbe Reaktoranordnung, derselbe Polymerisationsinhibitor, derselbe Katalysator (Art und Menge) und dasselbe Siedeöl (Art und Menge) verwendet. Bei stationären Bedingungen stellte sich ein Temperaturprofil von 167 °C (Sumpf) bis 23 °C (Kolonnenkopf) ein.

Es wurden 1500 g/h Essigsäureanhydrid und 2118 g/h Acrylsäure kontinuierlich frisch zudosiert.

Am Kolonnenkopf fielen 1712 g/h Essigsäure an. Am Seitenstromabzug wurden 1797 g/h Acrylsäureanhydrid mit einer Reinheit von 99,7 % (GC-Analyse) entnommen. Die Ausbeute an Acrylsäureanhydrid bezogen auf eingesetztes Essigsäureanhydrid bzw. eingesetzte Acrylsäure betrug 97 %.

## Patentansprüche

1. Verfahren zur kontinuierlichen Herstellung von ungesättigten Carbonsäureanhydriden der allgemeinen Formel I
R-C(O)-O-C(O)-R (I),
in der R einen ungesättigten organischen Rest mit 2 bis 12 C-Atomen darstellt, durch Umanhydridisierung von einem aliphatischen Carbonsäureanhydrid mit einer Carbonsäure der allgemeinen Formel II
R-COOH (II),
in der R die oben angegebene Bedeutung hat,
in einer Rektifikationskolonne mit einem oberen, mittleren und unteren Bereich, **dadurch gekennzeichnet, dass**
a) im Sumpf der Kolonne ein inertes Siedeöl vorgelegt ist,
b) die Edukte in stöchiometrischen Verhältnissen in einen Reaktionsbereich eingespeist werden,
c) am Kopf der Kolonne die als Nebenprodukt entstehende Carbonsäure entnommen wird,
d) die nicht umgesetzten Edukte in den Reaktionsbereich zurückgeführt werden und
e) das Produkt der Formel I über einen Seitenabzug, bevorzugt zwischen dem mittleren und unteren Kolonnenbereich, erhalten wird.

2. Verfahren gemäß Anspruch 1, **dadurch gekennzeichnet, dass** in dem Reaktionsbereich ein heterogener Katalysator verwendet wird.

3. Verfahren gemäß Anspruch 2, **dadurch gekennzeichnet, dass** ein saurer Festbettkatalysator verwendet wird.

4. Verfahren gemäß Anspruch 2 oder 3, **dadurch gekennzeichnet, dass** ein Kationenaustauscher als Katalysator verwendet wird.

5. Verfahren gemäß einem der Ansprüche 1 bis 4, **dadurch gekennzeichnet, dass** sich der Reaktionsbereich außerhalb der Kolonne befindet.

6. Verfahren gemäß einem der Ansprüche 1 bis5, **dadurch gekennzeichnet, dass** das ungesättigte Carbonsäureanhydrid der Formel I (Meth)acrylsäureanhydrid ist, hergestellt durch Umanhydridisierung von Essigsäureanhydrid und (Meth)acrylsäure.

7. Verfahren gemäß einem der Ansprüche 1 bis 6, **dadurch gekennzeichnet, dass** als Siedeöl eine hochsiedende, inerte Substanz mit einem Siedepunkt höher als die Siedepunkte der an der Reaktion beteiligten Komponenten verwendet wird.

8. Verfahren gemäß einem der Ansprüche 1 bis 7, **dadurch gekennzeichnet, dass** als Siedeöl 2,6-di-tert-Butyl-para-cresol, 2,6-di-tert-Butyl-phenol, Sulfolan oder Diphyl oder Mischungen aus diesen verwendet werden.

9. Verfahren gemäß einem der Ansprüche 1 bis 8, **dadurch gekennzeichnet, dass** als Siedeöl Sulfolan verwendet wird.

10. Verfahren gemäß einem der Ansprüche 1 bis 9, **dadurch gekennzeichnet, dass** aus dem Kolonnensumpf hochsiedende Komponenten ausgeschleust werden und verdampfende Stoffe in die Kolonne zurückgeführt werden.

## Claims

1. Process for continuously preparing unsaturated carboxylic anhydrides of the general formula I
R-C(O)-O-C(O)-R (I)
in which R is an unsaturated organic radical having 2 to 12 carbon atoms
by transanhydridization of an aliphatic carboxylic anhydride with a carboxylic acid of the general formula II
R-COOH (II)
in which R is as defined above
in a rectification column having an upper, middle and lower region, **characterized in that**
a) an inert boiling oil is initially charged in the bottom of the column,
b) the reactants are fed into a reaction region in stoichiometric ratios,
c) the carboxylic acid formed as the by-product is withdrawn at the top of the column,
d) the unconverted reactants are recycled into the reaction region and
e) the product of the formula I is obtained via a side draw, preferably between the middle and lower column region.

2. Process according to Claim 1, **characterized in that** a heterogeneous catalyst is used in the reaction region.

3. Process according to Claim 2, **characterized in that** an acidic fixed bed catalyst is used.

4. Process according to Claim 2 or 3, **characterized in that** a cationic exchanger is used as the catalyst.

5. Process according to one of Claims 1 to 4, **characterized in that** the reaction region is outside the column.

6. Process according to one of Claims 1 to 5, **characterized in that** the unsaturated carboxylic anhydride of the formula I is (meth)acrylic anhydride, prepared by transanhydridization of acetic anhydride and (meth)acrylic acid.

7. Process according to one of Claims 1 to 6, **characterized in that** the boiling oil used is a high-boiling inert substance having a boiling point higher than the boiling points of the components involved in the reaction.

8. Process according to one of Claims 1 to 7, **characterized in that** the boiling oil used is 2,6-di-tert-butyl-para-cresol, 2,6-di-tert-butylphenol, sulpholane or Diphyl or mixtures thereof.

9. Process according to one of Claims 1 to 8, **characterized in that** the boiling oil used is sulpholane.

10. Process according to one of Claims 1 to 9, **characterized in that** high-boiling components are discharged from the column bottom and evaporating substances are recycled into the column.

## Revendications

1. Procédé pour la production en continu d'anhydrides d'acides carboxyliques insaturés de formule générale I
R-C(O)-O-C(O)-R (I),
dans laquelle R représente un radical organique insaturé ayant de 2 à 12 atomes de carbone,
par trans-anhydrisation d'un anhydride d'acide carboxylique aliphatique avec un acide carboxylique de formule générale II
R-COOH (II)
dans laquelle R a la signification donnée ci-dessus,
dans une colonne de rectification comportant une zone supérieure, une zone médiane et une zone inférieure,
**caractérisé en ce que**
a) une huile bouillante inerte est disposée au pied de la colonne,
b) les produits de départ sont introduits en rapports stoechiométriques dans une zone de réaction,
c) l'acide carboxylique formé en tant que sous-produit est prélevé à la tête de la colonne,
d) les produits de départ n'ayant pas réagi sont renvoyés dans la zone de réaction et
e) le produit de formule I est obtenu via un conduit de décharge latéral, de préférence entre la zone médiane et la zone inférieure de la colonne.

2. Procédé selon la revendication 1, **caractérisé en ce qu'**on utilise un catalyseur hétérogène dans la zone de réaction.

3. Procédé selon la revendication 2, **caractérisé en ce qu'**on utilise un catalyseur acide en lit fixe.

4. Procédé selon la revendication 2 ou 3, **caractérisé en ce qu'**on utilise comme catalyseur un échangeur de cations.

5. Procédé selon l'une quelconque des revendications 1 à 4, **caractérisé en ce que** la zone de réaction se trouve à l'extérieur de la colonne.

6. Procédé selon l'une quelconque des revendications 1 à 5, **caractérisé en ce que** l'anhydride d'acide carboxylique insaturé de formule I est l'anhydride (méth)acrylique, produit par trans-anhydrisation d'anhydride acétique et d'acide (méth)acrylique.

7. Procédé selon l'une quelconque des revendications 1 à 6, **caractérisé en ce qu'**on utilise en tant qu'huile bouillante une substance inerte à haut point d'ébullition, ayant un point d'ébullition supérieur aux points d'ébullition des composants participant à la réaction.

8. Procédé selon l'une quelconque des revendications 1 à 7, **caractérisé en ce qu'**on utilise comme huile bouillante le 2,6-di-tert-butyl-para-crésol, le 2,6-di-tert-butyl-phénol, le sulfolane ou le Diphyl ou des mélanges de ceux-ci.

9. Procédé selon l'une quelconque des revendications 1 à 8, **caractérisé en ce qu'**on utilise comme huile bouillante le sulfolane.

10. Procédé selon l'une quelconque des revendications 1 à 9, **caractérisé en ce que** les composants à haut point d'ébullition sont évacués du pied de la colonne et les substances vaporisées sont renvoyées dans la colonne.
